# EUROPEAN PATENT APPLICATION

(11) **EP 0 555 797 A1**
(43) Date of publication of application: **18.08.1993**
(21) Application number: 93101922.8
(22) Date of filing: 08.02.1993
(51) Int. Cl.: C12N 9/22, C12Q 1/44, C12Q 1/68

(54) **General buffer for restriction endonucleases**

(30) Priority: 10.02.1992 US 833205
(71) Applicant: Becton Dickinson and Company, Franklin Lakes, New Jersey 07417-1880 (US)
(72) Inventor: Dey, Margaret S., Apex, North Carolina 27502 (US)
(74) Representative: Rambelli, Paolo

(57) **Abstract**

Restriction endonuclease buffer compositions which are suitable for use with essentially all restriction endonucleases. The compositions comprise Tris-HCl, MgCl₂, and at least one of KCl, beta-mercaptoethanol and deoxyribonucleoside triphosphate. The buffer compositions provide efficient restriction of DNA using a wide variety of restriction endonucleases, regardless of the specific buffer requirements of the restriction endonuclease when using conventional restriction buffers.

## Description

### FIELD OF THE INVENTION

The present invention relates to digestion of nucleic acids with restriction endonucleases and in particular to buffers used to perform such digestions.

### BACKGROUND OF THE INVENTION

Restriction endonucleases are a group of enzymes which cut or nick deoxyribonucleic acids (DNA), usually generating fragments of DNA of defined length and sequence. Restriction endonucleases generally recognize specific base pair sequences in double stranded DNA and cut the phosphodiester bonds at a defined site in each strand to generate fragments having either staggared ends with short single stranded tails or blunt ends which are completely double stranded.

Restriction endonucleases are used extensively in recombinant DNA technology to generate defined fragments of DNA for cloning. By knowing the location of various recognition sequences for restriction endonucleases in DNA, the desired fragment can be isolated, linked to other DNA fragments as needed and thereby isolated for further study.

In order to make cuts in the phosphodiester bonds of DNA, restriction endonucleases were thought to require specific, defined buffer conditions for each restriction endonuclease. The buffer conditions which give optimum cutting varied with the restriction endonuclease being used. Certain restriction endonucleases were believed to require conditions of low salt while others performed optimally under conditions of medium or high salt. Certain restriction endonucleases exhibited other requirements, such as for acetate buffer or particular cofactors.

Because different restriction endonucleases required different prior art buffers, certain combinations of restriction endonucleases were incompatible in multiple digests or their cutting efficiency was suboptimal when the multiple digest was performed as a single step in one of these prior art buffers. Methods for digestion of DNA with multiple restriction endonucleases have therefore customarily been performed as a series of single digests, changing the buffer conditions for each restriction endonuclease. That is, the multiple digestion is performed as a series of sequential steps, each involving a single endonuclease. As a consequence, such multiple restriction digests can be difficult and time consuming and are subject to loss of digested DNA at each step.

Many restriction endonuclease buffers, designed for use with specific restriction endonucleases, are known in the art. See, for example Maniatis et al. Molecular Cloning; Methods in Enzymology, Vol. 68; and biotechnology product catalogs such as those available from New England Biolabs (Beverly, Massachusetts), United States Biochemical Corp. (Cleveland, Ohio) and Stratagene (La Jolla, California). These sources describe restriction endonucleases, their recognition sequences in DNA and their buffer requirements.

The restriction endonuclease buffers previously known in the art are formulated to meet the specific requirements of different restriction endonucleases. They generally contain varying amounts of sodium chloride to accommodate the salt requirements of a given restriction endonuclease, or are formulated without sodium chloride for use with salt-inhibited restriction endonucleases. Certain restriction endonucleases require a buffer formulated with acetate rather than chloride salts. These buffers generally contain magnesium acetate, Tris-acetate and potassium acetate instead of magnesium chloride, Tris-HCl and potassium chloride. See, for example, the compositions of Stratagene's "Optimal Buffers" and New England Biolabs "NEBuffers" as described in their respective product catalogs.

Because of the need in the art to simplify restriction digestion of DNA, a "universal" restriction endonuclease buffer has been sought. Stratagene markets such a "universal buffer." It contains potassium acetate, Tris-acetate pH 7.6, magnesium acetate, β-mercaptoethanol and bovine serum albumin (BSA), but suffers from the drawback that the concentrated "universal buffer" must be diluted to different extents for use with different restriction endonucleases. Therefore, even with this "universal buffer" it is necessary to provide different reaction conditions to obtain optimal activity for different restriction endonucleases. In addition, the Stratagene "universal buffer" does not overcome the problem of how to perform simultaneous multiple digests using restriction endonucleases which have different buffer requirements.

The polymerase chain reaction (PCR) for amplification of DNA has provided a means for analysis of rare nucleotide sequences (K. B. Mullis and F. A. Faloona, Methods Enzymol. 155, 335 (1987); R. K. Saiki et al., Science 230, 1350 (1985); R. K. Saiki et al., Science 239, 487 (1988)). Many different buffers are used for PCR amplification of DNA and they contain some of the same components as restriction endonuclease buffers. See, for example, R. K. Saiki et al., Science 230, 1350 (1985); R. K. Saiki et al. Science 239, 487 (1988); V. Shyamala and G. F. Ames, Gene 97,1 (1991); D. De Wit et al., J. Clin. Microbiol. 28, 2437 (1990); A. Brisson-Noel et al., Lancet ??, 1069 (1989); J. W. U. Fries et al., Molec. Cell. Probes 4, 87 (1990); R. J. Patel, J. Clin. Microbiol. 28, 513 (1990); V. Sritharan and R. H. Barker, Molec. Cell. Probes 5, 385 (1991). However, PCR buffers known in the art often contain additional ingredients not commonly found in restriction endonuclease buffers, e.g., gelatin, dimethylsulfoxide, ammonium sulfate and deoxyribonucleoside triphosphates.

The present invention overcomes several problems in the art of restriction endonuclease digestion of DNA. A general, all-purpose restriction endonuclease buffer is provided which allows efficient cutting of DNA by essentially all restriction endonucleases. Because the buffer of the present invention provides suitable conditions for DNA cleavage by many restriction endonucleases, such cleavages may be performed singly or in combination without concern for buffer compatibility. That is, using the present inventive buffer, essentially all restriction endonucleases are compatible. Furthermore, the inventive restriction endonuclease buffer is used at the same concentration regardless of the restriction endonuclease being used and thus avoids the need to dilute a stock solution differently for each restriction endonuclease.

Availability of such an all-purpose restriction endonuclease buffer significantly simplifies restriction digestion of DNA and has the advantage of cost savings, since purchase of multiple specialized buffers can be avoided. Use of the inventive buffer also provides time savings, since digestion of DNA with multiple restriction endonucleases can be performed without additional steps to change the buffer for each enzyme. Avoidance of additional steps for changing buffers also improves recovery of the digested DNA by reducing loss of the product at each isolation and recovery step.

### SUMMARY OF THE INVENTION

The present invention provides a buffer for restriction endonuclease digestion of DNA which is compatible with a wide range of restriction endonucleases. The buffer therefore allows the practitioner to use a single buffer at a single concentration for restriction digests involving many different restriction endonucleases, even those which have otherwise incompatible buffer requirements, and still obtain efficient cleavage of DNA. The ability to use a single buffer for many restriction endonucleases therefore realizes a cost savings and obviates the need to recognize and provide for the specific buffer requirements of different restriction endonucleases.

The availability of a general all-purpose restriction endonuclease buffer provides a particular advantage when it is desired to perform a restriction digest involving several restriction endonucleases, especially when those restriction endonucleases have otherwise incompatible buffer requirements. Using the inventive buffer, such a multiple digest can be performed in a single step incorporating all of the desired restriction endonucleases at once. In this way, the need to perform each digest individually with a single restriction endonuclease and to change the buffer between steps is avoided, resulting in time and cost savings as well as improved recovery of the digested DNA.

The general, all-purpose restriction endonuclease buffer of the present invention comprises, at 1X concentration, approximately 1 - 5 mM magnesium chloride (MgCl₂), 10mM Tris-HCl, pH 8.8, and at least one of the following additional ingredients:
0 - 60 mM potassium chloride (KCl)
0 - 15 mM β-mercaptoethanol (βME)
0 - 600 µM deoxyribonucleoside triphosphates (dNTP's)
Also provided are methods for performing restriction endonuclease digestion of DNA using the general all-purpose restriction endonuclease buffer of the invention.

Also provided are kits for use in performing restriction endonuclease digestion of DNA which comprise one or more restriction endonucleases and the general all-purpose restriction endonuclease buffer of the invention.

### DESCRIPTION OF THE DRAWINGS

Fig. 1A is a photograph of lambda HindIII digested DNA run on an agarose gel after being restricted with AluI in the buffers of the invention. Lanes 1-8 represent digests performed using buffer compositions 1-8 shown in Table IV.

Fig. 1B is a photograph of lambda HindIII digested DNA run on an agarose gel after restriction with HincIII in the buffers of the invention. Lanes 1-8 represent digests performed using buffer compositions 1-8 shown in Table IV.

Fig. 2A is a photograph of lambda DNA run on an agarose gel after being restricted with DdeI in buffer compositions 21-28 shown in Table VI. In each composition, one or more components of the composition were left out to determine which ingredients were essential to obtain restriction of DNA.

Fig. 2B is the same as Fig. 2A, except that HaeIII was used to restrict the lambda DNA.

Fig. 2C is the same as Fig. 2A, except that HincII was used to restrict the lambda DNA.

### DETAILED DESCRIPTION OF THE INVENTION

The general, all-purpose restriction endonuclease buffer of the present invention can be used with essentially any restriction endonuclease to restrict DNA, thereby reducing the need to purchase and maintain a variety of specific buffer compositions designed for use with individual restriction endonucleases. The components of the all-purpose restriction endonuclease buffer at 1X concentration are 1) approximately 10mM Tris-HCl, pH 8.8, 2) 1 - 5 mM MgCl₂ and at least one of the following additional components: a) potassium chloride, b) β-mercaptoethanol and c) deoxyribonucleoside triphosphates.

MgCl₂ is believed to be the most important ingredient of the restriction endonuclease buffer. It appears to be required for restriction of DNA by all endonucleases tested. MgCl₂ is present in the compositions at a concentration of about 1 - 5 mM, preferably about 3 mM, but the exact concentration does not appear to be critical for cleavage of DNA to take place efficiently. The concentration of MgCl₂ can be adjusted to optimize for a particular restriction endonuclease using procedures within the ordinary skill in the art.

In addition to MgCl₂, the inventive compositions contain any or all of three additional ingredients. These ingredients are potassium chloride (KCl), β-mercaptoethanol (βME) and deoxyribonucleoside triphosphates (dNTP's). It does not appear to be critical which of these additional ingredients is present in addition to MgCl₂ to achieve restriction of DNA with any particular restriction endonuclease, but at least one of them is preferably included in the compositions in addition to the MgCl₂ and Tris-HCl. Which of the additional ingredients are included in the restriction endonuclease buffer can be selected by the practitioner based on analysis of the efficiency of cutting of DNA by the restriction endonuclease of interest in a particular application. Such screening involves only routine procedures which are within the ordinary skill in the art.

If KCl is included in the restriction endonuclease buffer, it will generally be present in a concentration of up to about 60 mM, preferably about 50 mM. KCl does not appear to be an absolute requirement for any particular restriction endonuclease, but for most restriction endonucleases cleavage of DNA is improved when KCl is included in the buffer compositions.

When βME is included in the restriction endonuclease buffer compositions, it is generally present at a concentration of up to about 15 mM, preferably about 10 mM. As for KCl, βME does not appear to be an absolute requirement of any particular restriction endonuclease, but its presence in the compositions improves cleavage of DNA by endonucleases generally.

When dNTP's are included in the restriction endonuclease buffer compositions, they will be present at a total concentration of up to about 600 µM, preferably about 500 µM. Examples of dNTP's suitable for use in the buffer compositions are deoxyadenosine triphosphate (dATP), deoxyguanosine triphosphate (dGTP), deoxycytidine triphosphate (dCTP), deoxythymidine triphosphate (dTTP) and combinations thereof. As with the other additional ingredients, dNTP's do not appear to be an absolute requirement of any particular restriction endonuclease but their presence in the buffer compositions generally improves cleavage of DNA by endonucleases.

Each of the foregoing additional ingredients may be present in the restriction endonuclease buffer compositions singly or in combination with one or more of the other additional ingredients described. Preferably, the buffer composition will comprise all of the additional ingredients in addition to MgCl₂ and Tris-HCl. Most preferably the 1X buffer comprises:
10 mM Tris-HCl, pH 8.8
3 mM MgCl₂
50 mM KCl
10 mM BME
500 µM dNTP's
As is the case for previously known restriction endonuclease buffers, the compositions of the present invention may be formulated as concentrated stock solutions for storage convenience. Preferably, the concentrated stock solution will be about a 10X concentrate which is diluted 1:10 in the restriction digest mixture at the time of use, resulting in a 1X buffer concentration in the restriction digest.

The restriction endonuclease buffers of the present invention may be used in DNA restriction digests using conventional methods. (Maniatis, et al. Molecular Cloning: A Laboratory Manual Cold Spring Harbor Laboratory Press, 1989.). In general the restriction digest comprises the DNA to be restricted, at least one restriction endonuclease and 1X all-purpose restriction endonuclease buffer. For most restriction endonucleases, the digest will be incubated at about 37^{o} C for about 1 hour. However, certain restriction endonucleases have higher or lower temperature optima . For example, BstEII, BstNI, TagI and ThaI have temperature optima of about 60 - 65^{o} C. and incubations involving these and other similar restriction endonucleases will generally be conducted at the higher temperature. PstI, for example, has a temperature optimum of about 21 - 37^{o} C, and digests involving this and other similar enzymes would be conducted at a lower temperature. Similarly, the length of time required to achieve the desired degree of digestion of the DNA will vary depending on whether a complete or partial digest is desired and the level of activity of the endonuclease involved. Appropriate adjustment of temperature, amount of endonuclease and incubation time to achieve the desired degree of digestion is routine in the art.

The buffer compositions of the present invention may be packaged, either as a 1X solution or a more concentrated formulation, in the form of a kit which further includes one or more restriction endonucleases for use with the buffer. Since the present invention allows a single buffer formulation to be used with essentially all restriction endonucleases, manufacturing costs and time can be reduced and the practitioner need not be concerned with matching the correct recommended buffer to a particular restriction endonuclease or determining the correct extent of dilution to achieve efficient restriction of DNA.

The compositions and methods set forth herein represent certain embodiments of the principles of the invention. It is to be understood that the present invention may be embodied in numerous alternative fashions and that such alternative embodiments may be conceived by those skilled in the art using only routine abilities and without departing from the spirit and scope of the present invention. Specific illustrative embodiments are set forth in the following Examples but are not to be considered as limiting the scope of the invention as defined by the appended claims.

### EXAMPLE 1

To demonstrate that the restriction endonuclease buffer of the present invention is compatible with restriction endonucleases having different prior art buffer requirements, the following restriction endonucleases were selected for testing:

| RE | Recommended Buffer |
|---|---|
| AluI | NEB #1 Low salt |
| BamHI | NEB BamHI + BSA |
| DdeI | NEB#3 High salt |
| EcoRI | NEB EcoRI |
| HaeIII | NEB #2 Medium salt |
| HincII | NEB #4 Acetate buffer |
| HindIII | NEB #2 Medium salt |
| RsaI | NEB #1 Low salt |
| MaeII | BRL High salt (50 mM Tris HCl; .22 M NaCl; 6 mM MgCl₂; 7 mM βME) |
| MboI | NEB #3 High salt |
| DpnI | NEB #4 Acetate buffer |

The enzymes and the buffers recommended for optimum activity of each were purchased from New England Biolabs (Beverly, Massachusetts). Digestion of DNA using the inventive buffer was compared to digestion using the specific buffer recommended by New England Biolabs for use with each endonuclease. All restriction buffers were diluted to 2X concentration from 10X stock solutions with sterile water. For each restriction endonuclease tested, two 0.5 ml tubes were prepared. One tube contained approximately 200-400 ng of lambda phage DNA (HindIII digested - Gibco BRL, Gaithersburg, Maryland), 10 µl of 2X all-purpose restriction endonuclease buffer (20 mM Tris-HCl, pH 8.8, 6 mM MgCl₂, 100 mM KCl, 20 mM BME, 100 mM dNTP's) and approximately 5-20 units (1 µl) of the selected restriction endonuclease. The other tube contained the same components but with 10 µl of 2X recommended restriction endonuclease buffer substituted for the all-purpose buffer. The control tube was made up without restriction endonuclease added.

The samples were incubated for 1 - 1.5 hour at 37 degrees C, then mixed with 3 µl of loading dye (25% Ficoll 400, .25% bromphenol blue, 25% xylene cyanol) and electrophoresed on a 1% agarose gel for about 20 min. at approximately 100V. The gel was stained with ethidium bromide, backstained, and photographed under ultraviolet light to visually assess the extent of digestion of the lambda DNA and the digestion pattern. The results of the visual evaluation of the gels are shown in Table I below:

**Table I**

| RE | | Extent of Digestion | |
|---|---|---|---|
| | | All-Purpose Buffer | Recommended Buffer |
| 1. | Control (No RE) | - | - |
| 2. | AluI | + | + |
| 3. | BamHI | - | - |
| 4. | DdeI | + | + |
| 5. | EcoRI | +/- | +/- |
| 6. | HaeIII | + | + |
| 7. | HincII | + | + |
| 8. | HindIII | - | - |
| 9. | RsaI | + | + |
| 10. | MaeII | + | +/- |
| 11. | MboI | + | + |
| 12. | DpnI | + | + |

For all restriction endonucleases tested, the extent and pattern of digestion products was the same when either the all-purpose buffer or the specific recommended buffer was used. This demonstrates that the all-purpose buffer of the invention allows digestion of DNA as efficiently as the specific recommended buffer for each endonuclease.

To confirm the results obtained with digestion of lambda DNA, the foregoing experiment was repeated using seven of the previous restriction endonucleases and substituting 10 µl (up to about 1 µg) of human DNA for the lambda phage DNA. Visual evaluation of the extent and pattern of digestion on the gels revealed the results shown in Table II:

**Table II**

| RE | Extent of Digestion | |
|---|---|---|
| | All-Purpose Buffer | Recommended Buffer |
| 1. Control (No RE) | - | - |
| 2. AluI | + | + |
| 3. BamHI | - | - |
| 4. DdeI | + | + |
| 5. EcoRI | +/- | +/- |
| 6. HaeIII | + | + |
| 7. HincII | +/- | +/- |
| 8. HindIII | +/- | +/- |

Again, for each restriction endonuclease the DNA was digested in a similar pattern and to a similar extent in either the all-purpose buffer of the invention or the specific buffer recommended for the endonuclease.

The experiment was repeated as described above for human DNA, substituting 10 µl (approximately 200-400 ng) of PhiX174 DNA (HaeIII digest - Gibco BRL, Gaithersburg, Maryland) in the digests. Again, the pattern and extent of digestion of the DNA was the same for each restriction endonuclease regardless of whether the all-purpose buffer or the specific recommended buffer was used. Visual evaluation of the gels revealed the results shown in Table III:

**Table III**

| RE | Extent of Digestion | |
|---|---|---|
| | All-Purpose Buffer | Recommended Buffer |
| 1. Control (No RE) | - | - |
| 2. AluI | + | + |
| 3. BamHI | - | - |
| 4. DdeI | + | + |
| 5 EcoRI | +/- | +/- |
| 6. HaeIII | - | - |
| 7. HincII | + | + |
| 8. HindIII | - | - |

The foregoing experiments show that a given restriction endonuclease will cleave various types of DNA equally well whether the general all-purpose restriction buffer of the invention or its own specifically formulated restriction buffer is used in the digest. For MaeII, better digestion was achieved using the all-purpose buffer than was obtained with the specific recommended restriction buffer. The results suggest that the inventive buffer can serve as a universal restriction buffer which is compatible with essentially all restriction endonucleases.

### EXAMPLE 2

To determine the functional range of concentrations for the components of the all-purpose restriction buffer, eight different formulations were evaluated with four different restriction endonucleases from New England Biolabs (AluI, DdeI, HaeIII and HincII). Each buffer was prepared as a 2X concentrate in 20 mM Tris-HCl pH 8.8. After dilution to 1X in the restriction digests, the concentrations of ingredients were as shown in Table IV:

**Table IV**

| Buffer # | KCl | MgCl2 | dNTP's | βME | H2O |
|---|---|---|---|---|---|
| 1 | 60mM | 5mM | 10µM | 5mM | 344µl |
| 2 | 25mM | 5mM | 60µM | 5mM | 404µl |
| 3 | 25mM | 1mM | 10µM | 5mM | 564µl |
| 4 | 25mM | 1mM | 60µM | 15mM | 284µl |
| 5 | 60mM | 5mM | 60µM | 15mM | 64µl |
| 6 | 60mM | 1mM | 10µM | 15mM | 224µl |
| 7 | 60mM | 1mM | 60µM | 5mM | 344µl |
| 8 | 25mM | 5mM | 10µM | 15mM | 284µl |

Four sets of the above eight buffers were prepared (one set for each restriction endonuclease being tested). To 10 µl of the 2X concentrate of each buffer was added 10 µl (about 200-400 ng) of lambda DNA (HindIII digested) to obtain a 1X concentration. One µl (about 5-20 units) of the desired restriction endonuclease was added to each of the eight buffer compositions. As a control, 10 µl of sterile water was added to one sample of each set instead of restriction endonuclease.

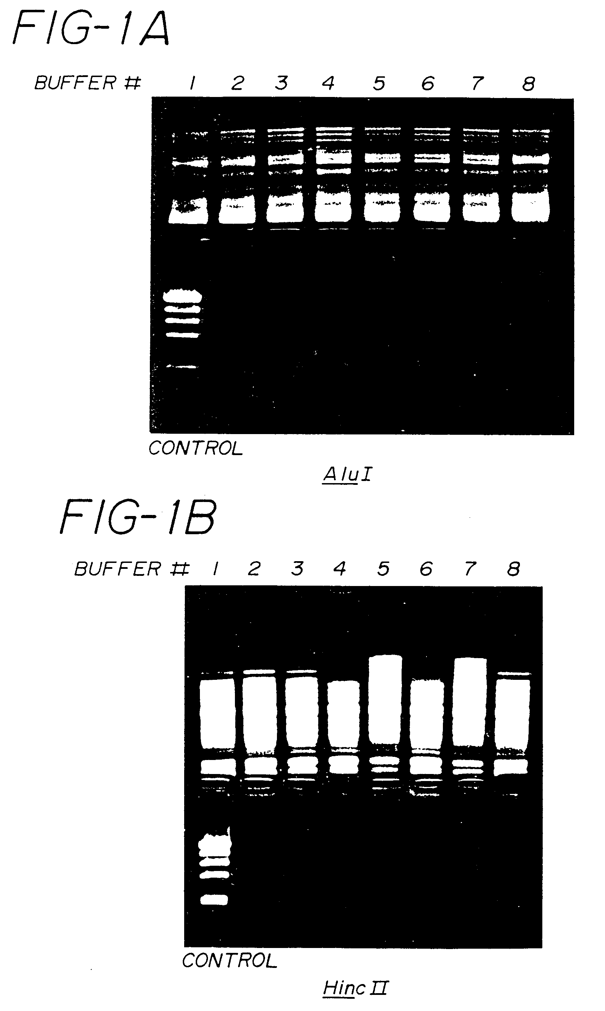

The digests were incubated at 37^{o} C for 1 hour. Each sample was then mixed with 2 µl of loading dye and run on a 1% agarose gel for about 20 min. at 100 V. The gels were stained with ethidium bromide for 20 min., backstained with sterile water for 20 min. and photographed under ultraviolet light using Polaroid film. As a representative example, the results for AluI are shown in Fig. 1A.

For AluI, DdeI and HaeIII there was no visually discernible difference in the extent or pattern of cleavage for any of the buffer compositions. As a more objective measure of the extent of digestion, the distance of the largest band from the well on the gel was measured for each buffer composition. The distance traveled by the largest fragment is affected by the size of the fragment, and the greater the degree of digestion, the smaller this fragment would be and the farther it would be expected to travel. For each of these three endonucleases the distance traveled by the largest fragment in the digest was found to be consistent regardless of the buffer composition tested. No digestion of DNA was visible in the control digests.

For HincII (Fig. 1B), all of the buffer compositions allowed some degree of cleavage of lambda DNA, but the distance traveled by the largest band on the gel was more variable than for the other three enzymes. These measurements are shown in Table V:

**Table V**

| Buffer # | Extent of Digestion (Visual) | Distance From Well (mm) |
|---|---|---|
| 1 | + | 14 |
| 2 | ++ | 18 |
| 3 | ++ | 18 |
| 4 | +++ | 21 |
| 5 | + | 14 |
| 6 | +++ | 20 |
| 7 | + | 14 |
| 8 | ++ | 18 |
| Control | - | 11 |

AluI, DdeI and HaeIII worked equally well in all buffer conditions. For HincII, cleavage of DNA may be more optimum at higher concentrations of βME and lower concentrations of KCl, dNTP and MgCl₂.

Shortening the incubation time for digestion to 20 minutes in a similar experiment using uncut lambda phage DNA (approximately 50 ng, unmethylated - Sigma Chemical Co., St. Louis, Missouri) also resulted in equivalent extent and pattern of cleavage for AluI and HaeIII. For DdeI, cleavage was similar for all buffer compositions except Buffer #3, which allowed only partial digestion in 20 minutes. For HincIII, Buffers # 3 and #5 allowed for the most complete cleavage. The remaining buffer compositions allowed partial cleavage in 20 minutes.

The foregoing results suggest that complex interactions between the components are involved. However, it is apparent that a wide range of concentrations of each component are possible without any significant negative effect on the use of the buffer composition for a wide variety of restriction endonucleases.

To determine which component or components of the all-purpose buffer were most important to the composition, a series of 2X buffers were formulated in 20mM Tris leaving out various components. When diluted 2:1 in the restriction digest mixtures, these buffers had the concentrations of components shown in Table VI:

**Table VI**

| Buffer # | KCl | MgCl2 | dNTP's | βME | H2O |
|---|---|---|---|---|---|
| 21 | 0mM | 3mM | 0µM | 10mM | 540µl |
| 22 | 50mM | 0mM | 0µM | 10mM | 400µl |
| 23 | 50mM | 3mM | 200µM | 10mM | 260µl |
| 24 | 0mM | 0mM | 200µM | 10mM | 520µl |
| 25 | 50mM | 0mM | 200µM | 0mM | 520µl |
| 26 | 50mM | 3mM | 0µM | 0mM | 540µl |
| 27 | 0mM | 0mM | 0µM | 0mM | 800µl |
| 28 | 0mM | 3mM | 200µM | 0mM | 660µl |

The digestion procedure was as previously described. AluI, DdeI, HaeIII and HincII were tested. As controls, endonuclease tested in its specific recommended buffer and uncleaved lambda DNA (approximately 50 ng, unmethylated - Sigma Chemical Co., St. Louis, Mo.) were included. Results were assessed visually on gels and by measuring the distance the largest lambda fragment had run.

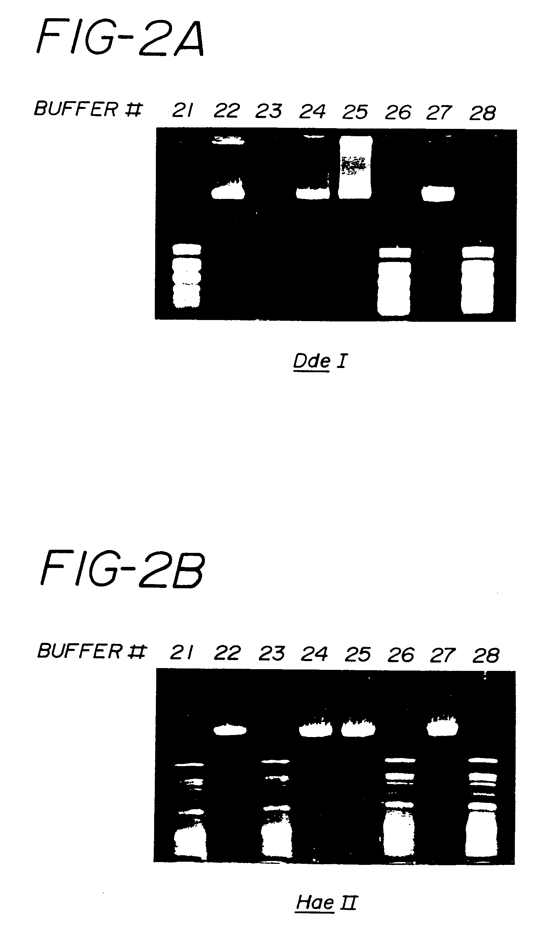

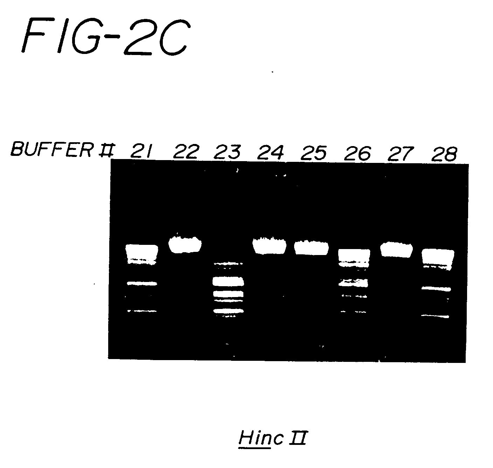

Cleavage of the DNA, in buffer compositions which allowed cleavage, was found to be equal to or better than cleavage in the specific recommended buffer except for HincII. In the case of HincII, cleavage in the specific recommended buffer was somewhat more complete but appeared to be less precise. That is, the cleavage products of HincII in the inventive buffer appeared as more distinct bands while those in the specific recommended buffer were more smeared. As examples of these test results, the gel of the DdeI digest is shown in Fig. 2A, the gel of the HaeIII digest is shown in Fig. 2B and the gel of the HincII digest is shown in Fig. 2C.

DNA was generally not digested in buffer compositions which lacked MgCl₂, suggesting that this component is the most important factor for DNA cleaving in the buffer compositions. However, complex interactions of components also appeared to be involved. Restriction endonucleases which normally have different salt requirements all digested the test DNA efficiently at the same concentration of MgCl₂ in the all-purpose buffer. In general, which additional ingredients were present in the buffer composition appeared to have little, if any effect on the efficiency of DNA cleavage.

## Claims

1. A restriction endonuclease buffer composition consisting essentially of:
a) about 10 mM Tris-HCl pH 8.8;
b) 1-5 mM MgCl₂; and
c) at least one additional ingredient selected from the group consisting of up to about 60 mM KCl, up to about 15 mM β-mercaptoethanol and up to about 600 µM deoxyribonucleoside triphosphates.

2. A restriction endonuclease buffer composition consisting essentially of:
a) 10 mM Tris-HCl;
b) 3 mM MgCl₂; and
c) at least one additional ingredient selected from the group consisting of 50 mM KCl, 10 mM β-mercaptoethanol and 50 µM deoxyribonucleoside triphosphates.

3. The composition according to Claim 2 consisting essentially of 10 mM Tris-HCl, 3 mM MgCl₂, 50 mM KCl, 10 mM β-mercaptoethanol and 500 µM deoxyribonucleoside triphosphates.

4. A buffer composition consisting essentially of Tris-HCl pH 8.8, MgCl₂, KCl, β-mercaptoethanol and deoxyribonucleoside triphosphates.

5. A 10X concentrated restriction endonuclease buffer composition consisting essentially of:
a) about 100 mM Tris-HCl pH 8.8;
b) 10-50 mM MgCl₂; and
c) at least one additional ingredient selected from the group consisting of up to about 600 mM KCl, up to about 150 mM β-mercaptoethanol and up to about 6 mM deoxyribonucleoside triphosphates.

6. The concentrated restriction endonuclease buffer composition according to Claim 5 consisting essentially of:
a) about 100 mM Tris-HCl pH 8.8;
b) 30 mM MgCl₂; and
c) at least one additional ingredient selected from the group consisting of 500 mM KCl, 100 mM β-mercaptoethanol and 5 mM deoxyribonucleoside triphosphates.

7. A method of restricting DNA with a restriction endonuclease comprising:
a) preparing a restriction digest comprising the DNA to be restricted, at least one restriction endonuclease and a buffer, said buffer consisting essentially of about 10 mM Tris-HCl pH 8.8, 1-5 mM MgCl₂ and at least one ingredient selected from the group consisting of up to about 60 mM KCl, up to about 15 mM β-mercaptoethanol and up to about 600 µM deoxyribonucleoside triphosphates; and
b) incubating the restriction digest under suitable conditions to allow cleavage of the DNA by the restriction endonuclease.

8. The method according to Claim 7 wherein the buffer is provided in a concentrated form which is diluted for preparation of the restriction digest.

9. The method according to Claims 7 or 8 wherein the restriction digest is prepared such that the buffer consists essentially of:
a) about 10 mM Tris-HCl pH 8.8;
b) 3 mM MgCl₂; and
c) at least one additional ingredient selected from the group consisting of 50 mM KCl, 10 mM β-mercaptoethanol and 500 µM deoxyribonucleoside triphosphates.

10. A kit for performing restriction digests of DNA comprising:
a) a 10X concentrated restriction endonuclease buffer contained in a first containing means, said buffer consisting essentially of about 100 mM Tris-HCl pH 8.8, 10-50 mM MgCl₂ and at least one ingredient selected from the group consisting of up to about 600 mM KCl, up to about 150 mM β-mercaptoethanol and up to about 6 mM deoxyribonucleoside triphosphates, and;
b) at least one restriction endonuclease contained in a second containing means, the restriction endonuclease being capable of cleaving the DNA in the presence of the buffer when the buffer is diluted to 1X concentration.
